# EUROPÄISCHE PATENTSCHRIFT

(11) **EP 1 262 162 B1**
(45) Veröffentlichungstag und Bekanntmachung des Hinweises auf die Patenterteilung: **19.11.2008**
(21) Anmeldenummer: 02011685.1
(22) Anmeldetag: 03.06.2002
(51) Int. Cl.: A61K 6/08, A61K 6/083

(54) **Farbstoffzusammensetzung zum individuellen Einfärben von Prothesenkunststoffen**
Dye composition for individual colouring of synthetic resin prostheses
Composition colorante pour la coloration de résines synthétiques pour prothèses

(30) Priorität: 01.06.2001 DE 10126968
(43) Veröffentlichungstag der Anmeldung: 04.12.2002
(73) Patentinhaber: Ivoclar Vivadent AG, 9494 Schaan (LI)
(72) Erfinder: Wachter, Wolfgang, 9494 Schaan (LI); Zanghellini, Gerhard, 9494 Schaan (LI); Rheinberger, Volker, 9490 Vaduz (LI); Kammann, Axel, 6800 Feldkirch (AT)
(74) Vertreter: UEXKÜLL & STOLBERG

(56) Entgegenhaltungen:
- EP-A- 0 836 845
- WO-A-01/08638
- WO-A-01/13814
- GB-A- 665 293
- US-A- 3 715 331
- US-A- 4 521 193
- US-A- 5 430 074
- US-B1- 6 224 799

## Beschreibung

Die Erfindung betrifft Farbstoffzusammensetzungen zur individuellen Einfärbung von Kunststoffen für dentale Prothesen.

In der Dentaltechnik ist es üblich, Restaurationen farblich individuell an die Mundraumsituation des Patienten anzupassen. Hierbei konzentriert man sich jedoch in erster Linie auf die Teile der Restauration, die die Zähne selbst betreffen. Zahnfleischfarbene Teile von Prothesen, wie beispielsweise Basisplatten von Vollprothesen, werden, wenn überhaupt, farblich nur grob dadurch auf den Patienten abgestimmt, daß der Zahntechniker aus einer vorgegebenen Farbpalette einen ihm geeignet erscheinenden Farbton auswählt.

Zur Herstellung von Basisplatten von Dentalprothesen werden heute meist Zweikomponentensysteme verwendet, die aus Polymerpulver und polymerisierbarem Monomer bestehen. Pulver und Monomer werden in einem vorgegebenen Verhältnis miteinander gemischt, in eine Gussform gefüllt und gehärtet. Das Polymerpulver ist eingefärbt und je nach Hersteller hat der Zahntechniker die Auswahl zwischen 4 bis 10 verschiedenen Farbtönen.

Die US 5,588,834 offenbart ein System zur farblichen Gestaltung von Dentalrestaurationen, das Farbmodifikationsmittel enthält, die neben Acrylsäuremonomeren und/oder -polymeren, mineralischen Füllstoffen und Photoinitiatoren Farbstoffe, wie beispielsweise Titandioxid, Eisenoxid, Chromverbindungen oder organische Farbstoffe, enthalten. Die Materialien werden mit einem Pinsel schichtweise auf Plastikkäppchen aufgetragen, die dann dem Zahntechniker als Vorlage zur Gestaltung der eigentlichen Restaurationen, wie Kronen und Brücken, dienen.

Aus der US 5,125,970 sind pulverförmige Farbmittel zur individuellen Gestaltung von Dentalrestaurationen bekannt, die Metalloxid enthalten und ggf. nach Zusatz eines Bindemittels mit einem Pinsel auf die Restauration aufgetragen werden.

Das Targis/Vectris-System der Firma Ivoclar Vivadent erlaubt in begrenztem Umfang eine individuelle Charakterisierung zahnfleischfarbener Teile von Dentalrestaurationen durch den schichtweisen Auftrag von Farben.

Die DE 196 35 667 C2 offenbart lichthärtende Opaker zur Abdeckung metallischer Oberflächen von Dentalrestaurationen. Die Opaker enthalten ein vernetztes Perlpolymerisat, in das ein farbiges Pigment eingearbeitet ist, und polymerisierbares polyfunktionelles Monomer.

Die GB 665,293 offenbart Zahnfleich farbene Dentalwerkstoffe auf der Basis von pinkfarbenen und ungefärbten, klaren Polymerpartikeln und flüssigem Monomer.

Die bekannten Materialien zur individuellen farblichen Gestaltung von Dentalrestaurationen werden schichtweise aufgetragen und sind für die individuelle Farbmodifikation zahnfleischfarbener Teile von Prothesen weniger geeignet, da hier eine durchgehende Einfärbung des Materials erwünscht ist. Materialien, die eine individuelle Einfärbung zahnfleischfarbener Teile von Prothesen erlauben, sind bisher nicht bekannt. Da die ästhetischen Ansprüche an Dentalrestaurationen allgemein gestiegen sind, besteht Bedarf an Materialien, die eine solche Einfärbung ermöglichen.

Der Erfindung liegt demzufolge die Aufgabe zugrunde, Dentalmaterialien bereit zu stellen, die die Herstellung individuell eingefärbter zahnfleischfarbener Dentalprothesen oder zahnfleischfarbener Teile von Dentalprothesen gestatten. Unter Einfärbung wird eine durchgehende, d.h. nicht nur oberflächliche Färbung des Materials verstanden.

Diese Aufgabe wird durch Materialien zur Herstellung von Dentalprothesen gelöst, die neben polymerisierbarem Matrixmaterial und Füllstoff mehrere Farbstoffzusammensetzungen enthalten. Diese Komponenten werden als Kit, d.h. in räumlich getrennter Form, vertrieben und erst vom Anwender miteinander gemischt.

Der Kit enthält mehrere unterschiedlich gefärbte Farbstoffzusammensetzungen, besonders bevorzugt mehrere Korrekturfarben und mindestens eine Grundfarbe, ganz besonders bevorzugt mehrere Korrekturfarben und mehrere Grundfarben. Die Grundfarbe wird so gewählt, daß sie dem gewünschten Farbton möglichst nahe kommt. Eine individuelle Abstimmung des Farbton erfolgt ggf. durch die Zugabe der Korrekturfarben.

Bei zahnfleischfarbenen Prothesen oder Prothesenteilen wird eine durchgehende Färbung angestrebt. Die homogene, schlierenfreie Einfärbung von Dentalmaterialien ist jedoch häufig mit Schwierigkeiten verbunden, da relativ geringe Farbstoffmengen in einem vertretbaren Zeitaufwand gleichmäßig in den Werkstoff eingearbeitet werden müssen. Erschwerend kommt hinzu, daß auf die dem Zahntechniker und Zahnarzt zur Verfügung stehenden Mischapparaturen zurückgegriffen werden soll, die der Leistungsfähigkeit industrieller Mischapparaturen nachstehen.

Erfindungsgemäß wird dieses Problem durch die Bereitstellung von Farbstoffzusammensetzungen gelöst, die auch in geringen Mengen eine homogene Verteilung in dem Werkstoff und damit eine gleichförmige Einfärbung gewährleisten, ohne den Mischaufwand im Vergleich zu herkömmlichen Zweikomponentensystemen zu erhöhen.

Die erfindungsgemäß verwendeten Farbstoffzusammensetzungen enthalten ein Bindemittel und mindestens ein organisches oder anorganisches Pigment.

Das oder die Pigmente werden nur oberflächlich an Polymerpartikel gebunden, beispielsweise indem Polymerpartikel und Pigment intensiv miteinander gemischt werden. Hierbei werden das oder die Pigmente physikalisch an die Oberfläche der Trägerpolymerpartikel gebunden. Die oberflächliche Bindung der Pigmente ermöglicht eine gleichmäßigere Pigmentverteilung im Matrixmaterial.

Zur Herstellung von Mischfarben können mehrere unterschiedlich gefärbte Pigmente gleichzeitig mit einem Trägerpolymer verarbeitet werden, vorzugsweise werden die Pigmente jedoch zunächst separat an Polymerteilchen gebunden und anschließend die unterschiedlich pigmentierten Polymerteilchen miteinander gemischt.

Als Trägerpolymere werden die in der Dentalindustrie gebräuchlichen Polymere, vorzugsweise Homo- und Copolymere auf der Basis von (Meth)acrylsäureestern, insbesondere Polymethyl(meth)acrylsäure- (PMMA)-Homopolymere und -Copolymere, verwendet. Vorzugsweise werden die gleichen Materialien eingesetzt, die auch im Matrixmaterial zur Anwendung kommen, so daß Eintrübungen, die bei der Verwendung unterschiedlicher Materialien möglich sind, vermieden werden und eine hohe Transparenz und Verträglichkeit der Materialien sichergestellt wird. Die Trägerpolymere sind vorzugsweise gesättigte Polymere, besonders solche mit einem Molekulargewicht von mehr als 50.000 g/mol.

Die mittlere Korngröße der Partikel des Trägerpolymers liegt zwischen 20 und 90 µm, wobei es sich als besonders vorteilhaft erwiesen hat, Partikel mit einer engen Korngrößenverteilung zu verwenden. Weiterhin sind kugelförmige Partikel bevorzugt. Besonders bevorzugt sind Polymerpartikel mit einer mittleren Korngröße von 40 bis 50 µm.

Perlpolymerisate, die sich z.B. auf an sich bekannte Weise durch Suspensionspolymerisation in wässriger Phase herstellen lassen, sind als Trägerpolymere besonders geeignet.

Die Trägerpolymere sind vorzugsweise frei von Initiatorresten, d.h. der Restgehalt an Peroxiden ist kleiner als 0,1 Gew.-%, um eine oxidative Zerstörung der Pigmente und damit mögliche Verfärbungen zu vermeiden.

Als Pigmente werden schwermetallfreie, d.h. insbesondere Cd-- und Pb-freie Pigmente verwendet. Die gebräuchlichsten anorganischen Pigmente sind solche auf der Basis der verschiedenen Eisenoxide, Chromate und Molybdate. Als organische Pigmente werden hauptsächlich Azopigmente, wie Monoazo-, Disazo-, Benzimidazolon- und Isoindolononpigmente, sowie polycyclische Pigmente, wie Phthalocyanin-, Thioindigo-, Flavanthron-, Dioxazin- und Anthantronpigmente, verwendet. Diese Substanzklassen werden durch die Verwendung von verschiedenen Substituenten hinsichtlich der Farbnuance und der Farbtiefe modifiziert. Herstellung, Anwendung und Eigenschaften der gebräuchlichsten organischen Pigmente werden ausführlich in Herbst/Hunger, "Industrielle Organische Pigmente", VCH-Verlagsgesellschaft, Weinheim, 1987 beschrieben.

Als Pigmente eignen sich besonders Ultramarinblau, Pigmente auf der Basis von Eisenoxid, Titandioxid, Kobalt-, Aluminium-, Chrom-, Nickel-, Zirkon- und/oder Zinkoxid, Ruß und organische farbige Pigmente. Weiterhin sind organische Pigmente, wie beispielsweise rote Diazokondensationspigmente, z.B. Microlith® rot BR-T (Fa. CIBA, Specialities) und gelbe Benzimidazolonpigmente, z.B. PV-Echtgelb H2G 01 (Fa. Hoechst), geeignet.

Die Eisenoxidpigmente können eine rote, gelbe, braune oder schwarze Farbe aufweisen.

Bevorzugte Pigmente sind schwarzes Eisenoxid, braunes Eisenoxid, gelbes organisches Pigment, rotes organisches Pigment und Titandioxid.

Der Pigmentgehalt der pigmentierten Polymerpartikel beträgt vorzugsweise 1 bis 15 Gew.-%, insbesondere 2 bis 12 Gew.-% bezogen auf die Gesamtmasse an Trägerpolymer und Pigment, wobei der Pigmentgehalt von der Farbintensität der einzelnen Pigmente abhängt. Im Fall von weißen Pigmenten beträgt er vorzugsweise 8 bis 12 Gew.-%, besonders bevorzugt etwa 10 Gew.-%, in allen anderen Fällen vorzugsweise 2 bis 4 Gew.-%, insbesondere 2,5 bis 3,5 Gew.-%. Die Pigmente können zusammen mit Hilfsstoffen, wie z.B. Talkum, auf das Trägerpolymer aufgebracht werden.

Die Farbstoff zusammensetzungen enthalten vorzugsweise 1 bis 80 Gew.-%, vorzugsweise 2 bis 70 Gew.-% pigmenthaltige Polymerpartikel, bezogen auf die Gesamtmasse der Farbstoffzusammensetzung.

Die Farbstoffzusammensetzung kann in Form einer fließfähigen oder pastenförmigen Dispersion oder in Form eines Presskörpers vorliegen. Unter Dispersionen werden fest-flüssig-Mehrphasensysteme verstanden, die eine im Dispersionsmedium unlösliche feste Komponente enthalten.

Zur Herstellung von Dispersionen werden die pigmenthaltigen Polymerpartikel mit einem flüssigen Bindemittel und gegebenenfalls weiteren Hilfsstoffen, wie Dispergierhilfsmitteln und rheologischen Additiven, versetzt und zu einer homogenen Masse gemischt. In Abhängigkeit vom Verhältnis von flüssigen zu festen Komponenten liegt die Dispersion entweder in Form einer fließfähigen oder pastenförmigen Masse vor.

Als Bindemittel zur Herstellung von Dispersionen sind solche Substanzen bevorzugt, die das Trägerpolymer anquellen. Unter Anquellen wird eine Oberflächenvergrößerung des Trägerpolymers durch das Bindemittel verstanden, die ein leichtes Ablösen der Pigmente beim späteren Mischen mit dem Matrixmaterial ermöglicht, was eine homogene Verteilung der Pigmente im Prothesenmaterial erleichtert. Durch das Anquellen der Trägerpolymerteilchen wird außerdem die Lagerstabilität der Dispersionen verbessert. Polymerisierbare Monomere sind als Bindemittel geeignet, jedoch auf Grund ihrer geringen Lagerstabilität nicht bevorzugt.

Besonders geeignete Bindemittel sind Phthalate und Citrate sowie deren Mischungen. Besonders bevorzugt ist Dibutylphthalat, insbesondere in Kombination mit Trägerpolymeren auf der Basis von (Meth)acrylsäureestern.

Übliche Hilfsmittel sind Dispergierhilfsmittel und rheologische Additive. Als rheologische Additive (Verdicker) sind hochdisperse Kieselsäure, Schichtsilikate, wie z.B. Bentone, hydrierte Rhizinusöle und Malonsäureester bevorzugt, als Dispergierhilfsmittel Talkum und Benetzungsmittel, wie z.B. Tenside.

Bevorzugte Farbstoffdispersionen weisen die folgende Zusammensetzung auf:

| pigmenthaltige | |
|---|---|
| Polymerpartikel | 8 bis 25 Gew.-%, vorzugsweise 10 bis 20 Gew.-%, besonders bevorzugt 12 bis 18 Gew.-%; |
| Bindemittel | 70 bis 92 Gew.-%, vorzugsweise 78 bis 89,5 Gew.-%, besonders bevorzugt 80 bis 87,5 Gew.-%; |
| Hilfsmittel | 0 bis 5 Gew.-%, vorzugsweise 0,5 bis 2 Gew.-%. |

Der Gesamtpigmentgehalt der Dispersionen liegt üblicherweise im Bereich von 0,1 bis 10 Gew.-%, vorzugsweise 0,15 bis 6 Gew.-%.

Zur Herstellung von Farbstoffzusammensetzungen in Form von Presskörpern werden die pigmenthaltigen Polymerpartikel vorzugsweise mit einem festen Bindemittel kombiniert. Im folgenden werden Farbstoffzusammensetzungen in Form von Presskörpern als tablettenförmige Farbstoffzusammensetzungen oder Tabletten bezeichnet. Auch hier kommt der Auswahl des Bindemittels eine besondere Bedeutung zu. Einerseits soll es den Zusammenhalt der Tabletten gewährleisten und einen vorzeitigen Zerfall beispielsweise beim Transport verhindern, andererseits aber beim Einarbeiten der Tablette in das Prothesenmaterial eine rasche und homogene Verteilung der Farbstoffpartikel sicherstellen.

Bevorzugte Bindemittel zur Herstellung von Tabletten sind Materialien mit Schmelzbereichen von 60 bis 100 °C, vorzugsweise 70 bis 90 °C und insbesondere etwa 85 °C, wie beispielsweise hydrierte Fettsäuren, z.B. Thixin ® E und Thixin® R (Fa. Elementis Specialities, Großbritannien), wachsartige methacrylierte Polyester, wie z.B. Uvecoat® 9010 (Fa. UCB, Belgien) und teilkristalline Polyester. Besonders bevorzugt sind Materialien mit einem Erweichungsbereich im Bereich von 35 bis 60 °C.

Unter dem Erweichungspunkt bzw. Erweichungsbereich versteht man die Temperatur oder den Temperaturbereich bei dem Gläser und amorphe und teilkristalline Polymere vom glasartigen oder hartelastischen in den gummielastischen Zustand übergehen. Der Erweichungspunkt liegt bei den meisten Polymeren deutlich unterhalb der Temperatur, bei der die Polymeren vollständig in den flüssigen Zustand übergehen.

Der Schmelzpunkt bzw. Schmelzbereich bezeichnet diejenige Temperatur, bei der die flüssige und die feste Phase eines Stoffes bei 1,013 bar Druck im thermodynamischen Gleichgewicht stehen. Bei amorphen, glasartigen Stoffen gibt es keine bestimmten Schmelzpunkt, da hier Kristallgitter fehlen.

Die Bindemittel weisen vorzugsweise ethylenisch ungesättigte Gruppen auf, die bei der Aushärtung des Prothesenmaterials durch chemische Bindungen in dieses eingebunden werden.

Das Bindemittel liegt bevorzugt in partikulärer Form vor, wobei die Partikelgröße vorzugsweise größer als die der pigmentierten Trägerpolymerteilchen ist. Bevorzugt sind partikuläre Bindemittel mit einer Korngröße von 100 bis 200 µm und insbesondere etwa 150 µm. Partikel aus den als Bindemittel bevorzugten Polymeren weisen eine wachsartige Oberfläche auf, in die die pigmentierten Trägerpolymerteilchen beim Pressen eingebettet werden. Auf diese Weise wird ein sicherer Zusammenhalt der Tabletten gewährleistet. Außerdem werden bei der Verwendung patikulärer Bindemittel Tabletten mit einer porösen Struktur erhalten, die das Eindringen des Matrixmaterials erleichtert und damit das Auflösen der Tablette und eine homogene Verteilung der pigmentierten Trägerpolymerteilchen und der Pigmente im Prothesenmaterial begünstigt.

Darüber hinaus können die tablettenförmigen Farbstoffzusammensetzungen auch anorganischen oder insbesondere organischen Füllstoff enthalten, wobei Polymerpartikel bevorzugt sind, die chemisch mit den Trägerpolymeren und dem Matrixmaterial verwandt sind. Die Füllstoffe werden vorzugsweise so gewählt, daß sie die Transparenz der Prothesenkunststoffe nicht beeinträchtigen.

Weiterhin können die Tabletten Additive und Hilfsmittel, beispielsweise Tablettierhilfsmittel, enthalten. Zur Beschleunigung der Auflösung der Tabletten können MBS-Modifier, wie Paraloid® 736 S (Fa. Röhm & Haas), zugegeben werden, die beim Kontakt mit dem Matrixmaterial quellen und dabei die Tablette sprengen. MBS ist das Kurzzeichen nach DIN 7728 T1, 1988 für Methacrylat/Butadien/Styrol-Copolymere.

Die bei der Herstellung von Tabletten oft als Presshilfsmittel eingesetzten Stearate sind als Additive ungeeignet, da sie einerseits die Handhabung der Tabletten beeinträchtigten, andererseits einen reproduzierbares und homogenes Einfärben der Prothesenmaterialien erschweren. Demzufolge enthalten die tablettenförmigen Farbstoffzusammensetzungen vorzugsweise keine Stearate.

Die Farbstoffzusammensetzungen, d.h. Dispersionen und Tabletten, enthalten vorzugsweise auch keine Initiatoren für die radikalische Polymerisation und insbesondere keine Peroxide, da diese durch Oxidation organischer Pigmente die Lagerstabilität der Mischungen verringern können.

Zur Herstellung von Tabletten werden pigmenthaltiges Trägerpolymer, Bindemittel und gegebenenfalls Füllstoff und Additive gemischt und anschließend zu Tabletten verpresst. Erfindungsgemäß werden unter Tabletten sowohl zylinderförmige Scheiben als auch würfelförmige, quaderförmige oder anders geformte Körper verstanden. Der Pressdruck wird so gewählt, daß die Tablette eine zur Handhabung ausreichende Festigkeit aufweist. Gemäß einer bevorzugten Ausführungsform wird die Tablette oberflächlich so geprägt oder mit Einkerbungen versehen, daß ein leichtes Teilen und Portionieren der Tablette möglich ist.

Nach dem Pressen können die Tabletten einer Nachbehandlung unterworfen werden, beispielsweise einem Temperschritt, um eine mechanische Verfestigung der Tabletten zu erreichen und ihre Handhabung zu verbessern. Das Tempern bewirkt ein Anschmelzen der Oberfläche des als Bindemittel verwendeten Polymers. Die Tabletten weisen vorzugsweise eine poröse Oberfläche auf, um so ein Eindringen von Matrixmaterial zu ermöglichen. Auf diese Weise wird ein schnelles Auflösen der Tablette und ein homogenes Verteilen der pigmentierten Polymerteilchen im Prothesenmaterial sichergestellt. Dieser Prozess kann durch ein leichtes Erwärmen des Prothesenmaterials begünstigt werden.

Bevorzugte tablettenförmige Farbstoffzusammensetzungen weisen die folgende Zusammensetzung auf:

| pigmenthaltige | |
|---|---|
| Polymerpartikel | 1 bis 70 Gew.-%, vorzugsweise 3 bis 60 Gew.-%, insbesondere 5 bis 50 Gew.-%; |
| Bindemittel | 5 bis 80 Gew.-%, vorzugsweise 10 bis 60 Gew.-%, insbesondere 15 bis 40 Gew.-%; |
| Füllstoff | 20 bis 80 Gew.-%, vorzugsweise 30 bis 70 Gew.-%, insbesondere 35 bis 65 Gew.-%: |
| Hilfsmittel | 0 bis 10 Gew.-%, vorzugsweise 0 bis 5 Gew.-%, insbesondere 0,5 bis 2 Gew.-%. |

Der Gesamtpigmentgehalt der Tabletten liegt üblicherweise im Bereich von 0,01 bis 10 Gew.-%, vorzugsweise 0,15 bis 6 Gew.-%.

Die erfindungsgemäßen Kits enthalten als polymerisierbares organisches Matrixmaterial vorzugsweise monofuktionelle oder polyfunktionelle (Meth)acrylate, die allein oder in Mischungen eingesetzt werden können. Als Matrixmaterial kommen beispielsweise Methylmethacrylat, Isobutylmethacrylat, Cyclohexylmethacrylat, Tetraethylenglycoldimethacrylat, Tri-ethylenglycoldimethacrylat, Diethylenglycoldimethacrylat, Ethylenglycoldimethacrylat, Polyethylenglycoldimethacrylat, Butandioldimethacrylat, Hexandioldimethacrylat, Decandioldimeth-acrylat, Dodecandioldimethacrylat, Bisphenol-A-dimethacrylat,Trimethylolpropantrimethacrylat, 2,2-Bis-[4-(2-Hydroxy-3-methacryloxypropoxy)-phenyl]-propan (Bis-GMA) sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tri-(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat, die als Urethandimethacrylate bezeichnet werden. Der Anteil dieser meist langkettigen Verbindungen im Matrixmaterial bewegt sich zwischen 10 und 80 Gew.-%.

Besonders bevorzugt sind Mischungen aus monofunktionellen (Meth)acrylaten und polyfunktionellen (Meth)acrylaten, wie beispielsweise Mischungen aus Methylmethacrylat, Ethylenglycoldimethacrylat und/oder Butandioldimethacrylat.

Als Füllstoff enthalten die Kits anorganischen oder vorzugsweise organischen Füllstoff, besonders bevorzugt Polymerpartikel auf der Basis der oben genannte Bindemittel, vorzugsweise Homo- und Copolymere auf der Basis von (Meth)acrylsäureestern, Polymethyl(meth)acrylsäure- (PMMA)-Homopolymeren und -Copolymeren. Die Füllstoffe weisen vorzugsweise eine Partikelgröße von 1 bis 200 µm, insbesondere 5 bis 100 µm auf.

Als Füllstoff können die bekannten, eingefärbten Polymerpulver verwendet werden. In diesem Fall enthält der Kit als Farbstoffzusammensetzung mindestens eine, vorzugsweise aber mehrere verschiedene Korrekturfarben. Kits, die ungefärbten Füllstoff und getrennte Grundfarben und Korrekturfarben enthalten, sind bevorzugt.

### Bevorzugte Kits enthalten:

| | |
|---|---|
| Matrixmaterial | 10 bis 80 Gew.-%, vorzugsweise 20 bis 70 Gew.-%, insbesondere 27 bis 55 Gew.-%; |
| Füllstoff | 20 bis 90 Gew.-%, vorzugsweise 30 bis 80 Gew.-%, insbesondere 45 bis 70 Gew.-%: |
| Farbstoffzusammensetzung | 0,2 bis 10 Gew.-%, vorzugsweise 0,5 bis 5 Gew.-%, insbesondere 0,7 bis 3 Gew.-%; |
| Hilfsmittel | 0 bis 10 Gew.-%, vorzugsweise 0 bis 5 Gew.-%, insbesondere 0 bis 2 Gew.-%. |

Zur Herstellung von Prothesen werden das polymerisierbare Matrixmaterial, Füllstoff und mindestens eine Farbstoffzusammensetzung miteinander gemischt und dann in eine geeignete Form gefüllt. Die anschließende Härtung des Materials erfolgt durch Hitze, Licht oder bei geeigneter Wahl des Initiatorsystems durch Mischen von Aktivator und Initiator (kalthärtendes System).

Die Aushärtung der Materialien erfolgt bevorzugt durch Heißhärtung unter Verwendung von Benzoylperoxid als Initiator. Weiterhin können mit den üblichen Initiator- und Aktivator-Systemen auf der Basis von Amin und Peroxid kalthärtende Systeme hergestellt werden. Bevorzugt sind Kombinationen von Benzoylperoxid mit tert-Aminen, wie Dimethyl-p-Toluidin, oder Barbitursäurederivate und Metallionen.

Gemäß einer bevorzugten Ausführungsform enthält der Kit zur Herstellung von Dentalprothesen mehrere Farbstoffzusammensetzungen, besonders bevorzugt mehrere Grundfarben und mehrere Korrekturfarben. Die Zusammensetzungen von Grund- und Korrekturfarben sind wie oben beschrieben, sie unterscheiden sich lediglich in Zahl und Menge der zugesetzten Pigmente.

Grundfarben enthalten in der Regel mehrere unterschiedlich gefärbte Pigmente, die erforderlich sind, um eine bestimmte Farbe zu erzielen. Zur Erzielung einer rosa- oder zahnfleischfarbenen Färbung werden vorzugsweise 4 bis 6 Pigmente verwendet. Eine besonders bevorzugte rosafarbene Farbstoffzusammensetzung enthält 5 Pigmente, nämlich weißes Pigment, vorzugsweise Titandioxid, schwarzes Pigment, vorzugsweise schwarzes Eisenoxid, braunes Pigment, vorzugsweise braunes Eisenoxid, gelbes Pigment, vorzugsweise PV-Echtgelb H2G 01 (Fa. Hoechst) und rotes Pigment, vorzugsweise Microlith® rot BR-T (Fa. Ciba Specialities). Durch Variationen der Mengen der einzelnen Pigmente lassen sich unterschiedliche rosa Grundtöne erzielen.

Die Korrekturfarben enthalten vorzugsweise nur 1 oder 2 verschieden gefärbte Pigmente, vorzugsweise nur 1 Pigment. Bevorzugt sind Korrekturfarben, die ein weißes, schwarzes, rotes oder braunes Pigment enthalten.

Vorzugsweise werden die einzelnen Pigmente separat an Trägerpolymerteilchen gebunden und dann die pigmentierten Trägerpolymere miteinander gemischt. Die Mischung wird anschließend zu einer Dispersion oder Tablette verarbeitet. Alternativ können die pigmenthaltigen Polymerteilchen auch getrennt zu Dispersionen geformt und erst dann miteinander gemischt werden.

Die erfindungsgemäßen Kits enthalten vorzugsweise 4 bis 6 Grundfarben. Der Pigmentgehalt und die Portionsgröße der Grundfarben werden vorzugsweise so bemessen, daß sie zur Einfärbung einer vorbestimmten Menge an Prothesenmaterial ausreichend sind, beispielsweise der Menge, die zur Herstellung einer Prothese benötigt wird. Bei Dispersionen kann die erforderliche Farbstoffmenge portionsweise in geeigneten Behältern oder aber in Dosierspendern, wie z.B. Dosierspritzen, verpackt werden, die die kontrollierte Entnahme einer bestimmten Menge erlauben. Bei tablettenförmigen Farbmischungen wird die Tablettengröße entsprechend gewählt oder die Tablette so gestaltet, daß eine problemlose Teilung entsprechend der gewünschten Portionsgröße möglich ist. Auf diese Weise können anhand eines Farbschlüssels die Mengen an Grund- und Korrekturfarbe ermittelt werden, die zur Erzielung des gewünschten Farbtons erforderlich sind, und eine kontrollierte und reproduzierbare Variation der Farbe wird ermöglicht.

Zur Herstellung von Dentalprothesen, wie beispielsweise der Basisplatte einer Vollprothese, können Matrixmaterial, Füllstoff und eine der Grundfarben miteinander gemischt und so ein Prothesenmaterial mit der gewünschten Grundfarbe erhalten werden.
Die Farbe des Prothesenmaterials kann anschließend durch die Zugabe von Korrekturfarben individuell angepasst werden. Vorzugsweise werden jedoch anhand eines Farbschlüssels vorab die erforderlichen Mengen an Grund- und Korrekturfarbe ermittelt und gleichzeitig mit dem Matrixmaterial und dem Füllstoff gemischt.

Unter einem Farbschlüssel wird eine Skala verstanden, die möglichst viele der Farbtöne aufweist, die mit dem jeweiligen System aus Grund- und Korrekturfarben erhältlich sind. Die Farbskala enthält vorteilhaft eine Vielzahl einzelner Farbelemente, die zur Farbbestimmung separat entnommen und z.B. an das Zahnfleisch des Patienten angehalten werden können. Die erfindungsgemäßen Kits enthalten vorzugsweise einen Farbschlüssel und eine Mischanweisung, die die zur Gewinnung des jeweiligen Farbtons erforderlichen Mengen an Grund- und Korrekturfarben angibt.

Die Kits enthalten bevorzugt 3 bis 10 verschiedene Korrekturfarben, besonders bevorzugt jeweils mindestens eine weiße, schwarze, rote und braune Korrekturfarbe.

Die erfindungsgemäßen Farbstoffzusammensetzungen lassen sich in die Materialien zur Herstellung der Dentalprothesen einarbeiten, ohne die bisher übliche Mischzeit zu verlängern.

Neben den genannten Komponenten können die erfindungsgemäßen Kits weitere optionale Komponenten zur Erzielung bestimmter Effekte enthalten, wie beispielsweise Kurzfasern oder Polymerkugeln. Kurzfasern, die bevorzugt aus Acetaten, PVC oder Cellulose bestehen, können lose oder in Tablettenform eingesetzt werden und dienen zur Erzielung von Anderungseffekten. Durch Zugabe von Polymerkugeln lässt sich die Transluzenz der Materialien einstellen. Bevorzugt sind Polymerkugeln aus vernetztem PMMA mit einer Größe von 20 bis 200 µm, vorzugsweise 30 bis 100 µm.

Gegenstand der Erfindung ist auch ein Verfahren zur Herstellung von Dentalprothesen, das dadurch gekennzeichnet ist, daß man
(i) ein Pigment oberflächlich an Polymerpartikel bindet, vorzugsweise oberflächlich auf die Partikel aufbringt,
(ii) man die pigmentbeschichteten Polymerpartikel mit einem Bindemittel kombiniert und zu einer fließfähigen oder pastenförmigen Dispersion oder zu einer Tablette formt;
(iii) man die Dispersion und/oder eine oder mehrere Tabletten und/oder Teile von Tabletten mit einem polymerisierbaren Matrixmaterial und Füllstoff mischt;
(iv) man die Mischung zu einer Dentalrestauration oder einem Teil davon formt und
(v) anschließend härtet.

Gemäß einer Variante des Verfahrens werden in Schritt (iii) das polymerisierbare Matrixmaterial und der Füllstoff zunächst mit einer ersten Farbstoffzusammensetzung gemischt und der Farbton der Mischung anschließend durch die Zugabe einer oder mehrerer weiterer Farbstoffzusammensetzungen abgestimmt, d.h. an einen gewünschten Farbton angepaßt. Vorzugsweise werden jedoch wie oben beschrieben vor dem Mischen die erforderlichen Mengen an Grundund Korrekturfarben ermittelt und gleichzeitig mit dem Matrixmaterial und dem Füllstoff gemischt.

Zur Herstellung von Dentalprothesen wird in der Regel zunächst die Patientensituation abgeformt (Funktionsabdruck) und dann ein Meistermodell (Gipsmodell) erstellt. Auf dem Modell wird mit Wachs die Prothesenbasis geformt und deren Paßform beim Patienten getestet (Funktionskontrolle). Danach wird das Wachsmodell in einer Küvette eingegipst, das Wachs ausgeschmolzen, der zurückbleibende Hohlraum mit dem Prothesenmaterial gefüllt und das Material anschließend gehärtet.

Unter Prothesen oder Dentalprothesen werden insbesondere Grundgerüste für dentale Restaurationen verstanden, auf denen Zähne aufgestellt werden, sowie Schutzprothesen, beispielsweise für den Sport.

### Beispiele

### Beispiel 1: Herstellung von Farbkonzentraten

In einem Intensiv-Pulvermischer wurden 88 Gew.-% eines Peripolymerisats auf der Basis von Methylmethacrylat (Plexidon® M 527, Firma Röhm, mittlere Korngröße ca. 50 µm) vorgelegt und mit 10 Gew.-% TiO₂ und 2 Gew.-% Talkum intensiv gemischt. Nach 10 min Mischzeit hatten sich das Titandioxid und das Talkum gleichmäßig auf das Polymerisat verteilt (Farbkonzentrat K1)

Zur Herstellung anderer Basis-Farbkonzentrate wurden getrennt jeweils 3 Gew.-% schwarzes Eisenoxid (K2), 3 Gew.-% braunes Eisenoxid (K3), 3 Gew.-% PV-Echtgelb H2G 01 (K4; gelbes Benzimidazolonpigment) und 2,5 Gew.-% des Rotpigments Microlith® BR-T (K5; rotes Diazokondensationspigment) mit 97 Gew.-% bzw. 97,5 Gew.-% Perlpolymerisat analog der oben beschriebenen Methode gemischt.

### Beispiel 2: Herstellung von Farbdispersionen (Korrekturfarben)

Zur Herstellung einer Farbdispersion wurden 84,5 g Dibutylphthalat auf 60 °C erwärmt und 15 g des Farbkonzentrats K1 aus Beispiel 1 und 0,5 g Bentone hinzugegeben und gerührt. Diese Mischung wurde bei 60 °C für 12 h stehen gelassen, um das Perlpolymerisat anzuquellen. Anschließend wurde die Mischung intensiv für 15 min gerührt und auf Raumtemperatur abgekühlt. Die so erhaltene Dispersion (D1) war gebrauchsfertig und konnte sofort in Dosierspritzen oder ähnlich geeignete Behältnisse verpackt werden.

Analog wurden auf der Basis der Farbkonzentrate K2 bis K5 die Korrekturfarben D2 bis D5 hergestellt.

### Beispiel 3: Herstellung einer Dispersion (Grundfarbe pink)

Zur Herstellung einer pinkfarbenen Dispersion wurden die Dispersionen D1 bis D5 in den nachstehend angegebenen Mengen durch einfaches Rühren miteinander gemischt:

| | | |
|---|---|---|
| D1 | (weiß) | 35,7 Gew.-% |
| D2 | (schwarz) | 6,1 Gew.-% |
| D3 | (braun) | 41,4 Gew.-% |
| D4 | (gelb) | 1,1 Gew.-% |
| D5 | (rot) | 15,7 Gew.-% |

Die so erhaltene Mischung konnte in Dosierspritzen oder ähnliche Behältnisse abgefüllt werden und war gebrauchsfertig.

### Beispiel 4: Einfärben eines ungefärbten Prothesenkunstoffmaterials

Zur Herstellung eines eingefärbten Prothesenkunststoffs wurden 69 Gew.-% PMMA-Füllstoff und 29 Gew.-% Matrixmaterial auf der Basis von Methylmethacrylat (Monomer) und Butandioldimethacrylat (Vernetzer) (Probase® Hot Clear, Fa. Ivoclar Vivadent AG) in einem geeigneten Behälter mit 2 Gew.-% der pinkfarbenen Dispersion gemäß Beispiel 3 durch einfaches Unterrühren mit einem Spatel eingefärbt und zu einem Prüfkörper geformt. Dieser wurde durch Erhitzen auf 100 °C innerhalb von 20 gehärtet. Der so hergestellte Prüfkörper wies eine homogene Pigmentverteilung auf und war absolut schlierenfrei.

### Beispiel 5: Herstellung von tablettenförmigen Farbstoffzusammensetzungen (Korrekturfarbe schwarz)

Zur Herstellung von Tabletten der Korrekturfarbe schwarz wurden 65 Gew.-% Perlpolymerisat auf der Basis von Methylmethacrylat (Plexidon® M 527, Firma Röhm, mittlere Korngröße ca. 50 µm) als Füllstoff mit 30 Gew.-% Polyester (Uvecoat® 9010, Fa. UCB, Korngröße < 160 µm, Erweichungsbereich 70 bis 90 °C) und 5 Gew-% des Farbkonzentrats K2 aus Beispiel 1 gemischt und bei einem Druck von 80 bar zu Tabletten mit einer Masse von jeweils 0,35 g verpresst. Anschließend wurden die Tabletten durch kurzzeitiges Tempern (15 Minuten bei 100 °C) verfestigt. Der Preßstempel war hierbei so geformt, daß die Tabletten einseitig Bruchkerben aufwiesen, so daß sie zur Portionierung leicht in vier Teile gebrochen werden konnten.

### Beispiel 6: Herstellung von pinkfarbenen Farbtabletten (Grundfarbe pink)

Zur Herstellung pinkfarbener Farbtabletten wurden die nachfolgend aufgeführten Komponenten in den angegebenen Mengen in einem Pulvermischer gegeben und für 10 Minuten bei Raumtemperatur intensiv gemischt.

| | | |
|---|---|---|
| K1 | (weiß) | 17,5 Gew.-% |
| K2 | (schwarz) | 3,0 Gew.-% |
| K3 | (braun) | 20,4 Gew.-% |
| K4 | (gelb) | 0,5 Gew.-% |
| K5 | (rot) | 8,0 Gew.-% |
| Polyester¹⁾ | | 14,3 Gew.-% |
| Perlpolymerisat²⁾ | | 36,3 Gew.-% |

| | | |
|---|---|---|
| ¹⁾ UVECOAT® 9010, Firma UCB, Korngröße < 160 µm, Schmelzbereich 70 bis 90 °C ²⁾ Plexidon® MW 527, Firma Röhm, mittlere Korngröße 50 µm | | |

Jeweils 0,35 g dieser Mischung wurden in einer gebräuchlichen Tablettenpresse zu Farbtabletten verpresst. Die Pressaufnahmen der Tablettenpresse waren so gestaltet, daß die Tabletten einseitig Bruchkerben aufwiesen, so daß zur definierten Einstellung der Farbtöne die Tablette in vier Teile geteilt werden konnte.

### Beispiel 7: Einfärben von Prothesenkunsstoff

Zur Herstellung eines gefärbten Prothesenkunststoffs wurden 24,5 g PMMA-Füllstoff und 25,1 g Matrixmaterial (SR-Ivocap® Clear, Fa. Ivoclar Vivadent AG), 1 Tablette der Grundfarbe pink gemäß Beispiel 6 und 1/4 Tablette der Korrekturfarbe schwarz gemäß Beispiel 5 in der Mischkapsel eines Rüttelmischers (Cap-Vibrator® , Firma Ivoclar Vivadent AG) für 5 min bei Raumtemperatur gemischt und analog Beispiel 4 ein Prüfkörper hergestellt und gehärtet. Der Prüfkörper wies eine homogene Pigmentverteilung auf und war absolut schlierenfrei.

## Patentansprüche

1. Kit zu Herstellung von Dentalprothesen, der
(i) polymerisierbares Matrixmaterial,
(ii) Füllstoff und
(iii) mehrere unterschiedlich gefärbte Farbstoffzusammensetzungen, die Polymerpartikel mit einer mittleren Korngröße von 20 bis 90 µm Bindemitell und mindestens ein schwermetallfreies Farbpigment enthalten, das oberflächlich an die Polymerpartikel gebunden ist,
in räumlich getrennter Form enthält.

2. Kit nach Anspruch 1, **dadurch gekennzeichnet, daß** die Farbstoffzusammensetzung als Polymerpartikel ein Perlpolymerisat enthält.

3. Kit nach einem der Ansprüche 1 bis 2, **dadurch gekennzeichnet, daß** die Farbstoffzusammensetzung Polymerpartikel auf der Basis von (Meth) acrylsäureestern enthält.

4. Kit nach einem der Ansprüche 1 bis 3, **dadurch gekennzeichnet, daß** die Polymerpartikel eine mittlere Korngröße von 40 bis 50 µm aufweisen.

5. Kit nach einem der Ansprüche 1 bis 4, **dadurch gekennzeichnet, daß** das Farbpigment Ultramarinblau, Eisenoxid, Titandioxid, ein farbiges Pigment auf der Basis von Kobalt-, Aluminium-, Chrom-, Nickel-, Zirkon- und/oder Zink-Oxid, Ruß und/oder ein organisches farbiges Pigment ist.

6. Kit nach einem der Ansprüche 1 bis 5, **dadurch gekennzeichnet, daß** die pigmenthaltigen Polymerpartikel 2 bis 15 Gew.% Farbpigment(e) bezogen auf die Gesamtmasse an Polymerpartikeln und Farbpigmenten enthalten.

7. Kit nach einem der Ansprüche 1 bis 6, **dadurch gekennzeichnet, daß** die Farbstoffzusammensetzung ein flüssiges Bindemittel enthält.

8. Kit nach Anspruch 7, **dadurch gekennzeichnet, daß** das Bindemittel so gewählt ist, daß es die Polymerpartikel anquillt.

9. Kit nach Anspruch 8, **dadurch gekennzeichnet, daß** das Bindemittel Dibutylphthalat ist.

10. Kit nach einem der Ansprüche 1 bis 9, **dadurch gekennzeichnet, daß** die Farbstoffzusammensetzung in Form einer fließfähigen oder pastenförmigen Dispersion vorliegt.

11. Kit nach einem der Ansprüche 1 bis 6, **dadurch gekennzeichnet, daß** die Farbstoffzusammensetzung ein festes Bindemittel mit einem Schmelzpunkt von 60 °C bis 100 °C enthält.

12. Kit nach einem der Ansprüche 7 bis 11, **dadurch gekennzeichnet, daß** das Bindemittel ethylenisch ungesättigte Gruppen aufweist.

13. Kit nach einem der Ansprüche 11 bis 12, **dadurch gekennzeichnet, daß** das Bindemittel ein teilkristalliner Polyester ist.

14. Kit nach einem der Ansprüche 11 bis 13, **dadurch gekennzeichnet, daß** das Bindemittel in partikulärer Form vorliegt.

15. Kit nach Anspruch 14, **dadurch gekennzeichnet, daß** das Bindemittel eine Partikelgröße von 100 bis 200 µm aufweist.

16. Kit nach einem der Ansprüche 11 bis 15, **dadurch gekennzeichnet, daß** die Farbstoffzusammensetzung zusätzlich Füllstoff enthält.

17. Kit nach Anspruch 16, **dadurch gekennzeichnet, daß** die Farbstoffzusammensetzung als Füllstoff Polymerpartikel auf der Basis von (Meth)acrylsäureestern enthält.

18. Kit nach einem der Ansprüche 11 bis 17, **dadurch gekennzeichnet, daß** die Farbstoffzusammensetzung zu einer Tablette geformt ist.

19. Kit nach einem der Ansprüche 1 bis 18, **dadurch gekennzeichnet, daß** die Farbstoffzusammensetzung 1 bis 80 Ges.-% pigmenthaltige Polymerpartikel bezogen auf die Gesamtmasse der Farbstoffzusammensetzung enthält.

20. Kit nach einem der Ansprüche 1 bis 19, **dadurch gekennzeichnet, daß** er mehrere Farbstoffzusammensetzungen in Form von Tabletten und/oder Dispersionen enthält, die jeweils mehrere Farbpigmente enthalten können.

21. Kit nach einem der Ansprüche 1 bis 20, **dadurch gekennzeichnet, daß** er einen Farbschlüssel und eine Mischanweisung enthält.

22. Kit nach einem der Ansprüche 14 bis 21, bei dem die Farbstoffzusammensetzung in Form eines Presskörpers vorliegt, der mindestens ein Farbpigment, das an Polymerpartikel gebunden ist, partikuläres Bindemittel und Füllstoff enthält.

23. Kit nach Anspruch 11, bei dem die Farbstoffzusammensetzung in Form einer Paste oder fließfähigen Dispersion vorliegt, die mindestens ein Farbpigment, das an Polymerpartikel gebunden ist, Bindemittel und Füllstoff enthält.

24. Verfahren zur Herstellung von Dentalprothesen, **dadurch gekennzeichnet, daß** (i) man ein gefärbtes Pigment oberflächlich auf Polymerpartikel mit einer mittleren Korngröße von 20 bis 90 µm aufbringt, (ii) man die pigmentbeschichteten Polymerpartikel mit einem Bindemittel kombiniert und zu einer fließfähigen oder pastenförmigen Dispersion oder zu einer Tablette formt, (iii) man zunächst anhand eines Farbschlüssels die erforderlichen Mengen Farbstoffzusammensetzungen ermittelt und man dann eine oder mehrere Dispersionen und/oder eine oder mehrere Tabletten und/oder Teile von Tabletten mit einem polymerisierbarem Matrixmaterial und Füllstoff mischt, (iv) man die Mischung zu einer Dentalrestauration oder einem Teil davon formt und (v) anschließend härtet.

25. Verfahren nach Anspruch 24, **dadurch gekennzeichnet, daß** man in Schritt (iii) das polymerisierbare Matrixmaterial und den Füllstoff zunächst mit einer ersten Farbstoffzusammensetzung mischt und man den Farbton der Mischung anschließend durch die Zugabe einer oder mehrerer weiterer Farbstoffzusammensetzungen abstimmt.

26. Verwendung einer Farbstoffzusammensetzung gemäß einem der Ansprüche 1 bis 232-4 zur individuellen Einfärbung von dentalen, zahnfleischfarbenen, füllstoffhaltigen Prothesen oder Prothesenteilen.

## Claims

1. Kit for preparing dentures which comprises
(i) polymerisable matrix material,
(ii) filler and
(iii) a plurality of differently coloured dyestuff compositions that comprise polymer particles with a mean particle size of 20 to 90 µm, binders and at least one heavy metal-free colour pigment that is superficially bound to the polymer particles,
in spatially separated form.

2. Kit according to Claim 1, wherein the dyestuff composition comprises a bead polymer as the polymer particle.

3. Kit according to Claim 1 to 2, wherein the dyestuff composition comprises a polymer particle based on (meth)acrylic acid esters.

4. Kit according to one of claims 1 to 3, wherein the polymer particles exhibit a mean particle size of 40 to 50 µm.

5. Kit according to one of Claims 1 to 4, wherein the colour pigment is ultramarine blue, iron oxide, titanium dioxide, a coloured pigment based on cobalt-, aluminium-, chromium-, nickel-, zirconium- and/or zinc oxide, carbon black and/or is an organic coloured pigment.

6. Kit according to one of Claims 1 to 5, wherein the pigment-containing polymer particles comprise 2 to 15 wt.% colour pigment(s), based on the total weight of polymer particles and colour pigments.

7. Kit according to one of Claims 1 to 6, wherein the dyestuff composition comprises a liquid binder.

8. Kit according to Claim 7, wherein the binder is selected such that it swells up the polymer particles.

9. Kit according to Claim 8, wherein the binder is dibutyl phthalate.

10. Kit according to one of Claims 1 to 9, wherein the dyestuff composition is in the form of a free-flowing or pasty dispersion.

11. Kit according to one of Claims 1 to 6, wherein the dyestuff composition comprises a solid binder with a melting point from 60 °C to 100 °C.

12. Kit according to one of Claims 7 to 11, wherein the binder possesses ethylenically unsaturated groups.

13. Kit according to one of Claims 11 to 12, wherein the binder is a partially crystalline polyester.

14. Kit according to one of Claims 11 to 13, wherein the binder is in particulate form.

15. Kit according to Claim 14, wherein the binder has a particle size of 100 to 200 µm.

16. Kit according to one of Claims 11 to 15, wherein the dyestuff composition additionally comprises filler.

17. Kit according to Claim 16, wherein the dyestuff composition comprises polymer particles based on (meth)acrylic acid esters.

18. Kit according to one of Claims 11 to 17, wherein the dyestuff composition is formed into a tablet.

19. Kit according to one of Claims 1 to 18, wherein the dyestuff composition comprises 1 to 80 wt.% pigment-containing polymer particles, based on the total weight of the dyestuff composition.

20. Kit according to one of Claims 1 to 19, comprising a plurality of dyestuff compositions in the form of tablets and/or dispersions, which can each comprise a plurality of colour pigments.

21. Kit according to one of Claims 1 to 20, comprising a colour key and mixing instructions.

22. Kit according to one of Claims 14 to 21, wherein the dyestuff composition is in the form of a moulded body that comprises a colour pigment bound to polymer particles, particulate binder and filler.

23. Kit according to Claim 11, wherein the dyestuff composition is in the form of a paste or free-flowing dispersion, which comprises at least one colour pigment bound to polymer particles, binder and filler.

24. Method for preparing dentures, wherein (i) a coloured pigment is superficially applied to polymer particles having a mean particle size of 20 to 90 µm, (ii) the pigment-coated polymer particles are combined and moulded into a free-flowing or pasty dispersion or into a tablet, (iii) the required amount of dyestuff composition is first determined using a colour key, and then one or a plurality of dispersions and/or one or a plurality of tablets and/or parts of tablets are mixed with a polymerisable matrix material and filler, (iv) the mixture is moulded into a dental restoration or a part thereof and (v) subsequently cured.

25. Method according to Claim 24, wherein in step (iii) the polymerisable matrix material and the filler are initially mixed and the colour tint of the mixture is then adjusted by adding one or a plurality of additional dyestuff compositions.

26. Use of a dyestuff composition according to one of Claims 1 to 23 for the individual colouring of gum-coloured, filler-containing dentures or parts of dentures.

## Revendications

1. Trousse pour la fabrication de prothèses dentaires, qui contient :
(i) un matériau de matrice polymérisable,
(ii) une charge, et
(iii) plusieurs compositions colorantes de teintes différentes, qui contiennent des particules polymères ayant une taille granulaire moyenne de 20 à 90 µm, un liant et au moins un pigment coloré exempt de métaux lourds, qui est lié en surface aux particules polymères.
sous une forme séparée spatialement.

2. Trousse selon la revendication 1, **caractérisée en ce que** la composition colorante contient, comme particules polymères, un polymère sous forme de perles.

3. Trousse selon l'une quelconque des revendications 1 et 2, **caractérisée en ce que** la composition colorante contient des particules polymères à base d'esters d'acide (méth)acrylique.

4. Trousse selon l'une quelconque des revendications 1 à 3, **caractérisée en ce que** les particules polymères présentent une taille granulaire moyenne de 40 à 50 µm.

5. Trousse selon l'une quelconque des revendications 1 à 4, **caractérisée en ce que** le pigment coloré est le bleu outremer, l'oxyde de fer, le dioxyde de titane, un pigment coloré à base d'oxyde de cobalt, d'aluminium, de chrome, de nickel, de zirconium et/ou de zinc, la suie et/ou un pigment coloré organique.

6. Trousse selon l'une quelconque des revendications 1 à 5, **caractérisée en ce que** les particules polymères contenant un pigment contiennent 2 à 15 % en poids de pigment(s) coloré(s) par rapport à la masse totale de particules polymères et de pigments colorés.

7. Trousse selon l'une quelconque des revendications 1 à 6, **caractérisée en ce que** la composition colorante contient un liant liquide.

8. Trousse selon la revendication 7, **caractérisée en ce que** le liant est choisi de manière qu'il permette la dilatation des particules polymères.

9. Trousse selon la revendication 8, **caractérisée en ce que** le liant est le phtalate de dibutyle.

10. Trousse selon l'une quelconque des revendications 1 à 9, **caractérisée en ce que** la composition colorante se présente sous la forme d'une dispersion fluide ou d'une dispersion pâteuse.

11. Trousse selon l'une quelconque des revendications 1 à 6, **caractérisée en ce que** la composition colorante contient un liant solide ayant un point de fusion de 60 °C à 100 °C.

12. Trousse selon l'une quelconque des revendications 7 à 11, **caractérisée en ce que** le liant présente des groupements éthyléniques insaturés.

13. Trousse selon l'une quelconque des revendications 11 et 12, **caractérisée en ce que** le liant est un polyester partiellement cristallin.

14. Trousse selon l'une quelconque des revendications 11 à 13, **caractérisée en ce que** le liant se présente sous une forme particulaire.

15. Trousse selon la revendication 14, **caractérisée en ce que** le liant présente une taille particulaire de 100 à 200 µm.

16. Trousse selon l'une quelconque des revendications 11 à 15, **caractérisée en ce que** la composition colorante contient également une charge.

17. Trousse selon la revendication 16, **caractérisée en ce que** la composition colorante contient, comme charge, des particules polymères à base d'esters d'acide (méth)acrylique.

18. Trousse selon l'une quelconque des revendications 11 à 17, **caractérisée en ce que** la composition colorante est élaborée sous la forme d'un comprimé.

19. Trousse selon l'une quelconque des revendications 1 à 18, **caractérisée en ce que** la composition colorante contient 1 à 80 % en poids de particules polymères contenant un pigment, par rapport à la masse totale de la composition colorante.

20. Trousse selon l'une quelconque des revendications 1 à 19, **caractérisée en ce qu'**elle contient plusieurs compositions colorantes sous la forme de comprimés et/ou de dispersions, qui peuvent contenir, respectivement, plusieurs pigments colorés.

21. Trousse selon l'une quelconque des revendications 1 à 20, **caractérisée en ce qu'**elle contient un nuancier et des instructions de mélange.

22. Trousse selon l'une quelconque des revendications 14 à 21, dans laquelle la composition colorante se présente sous la forme d'un comprimé, qui contient au moins un pigment coloré qui est lié aux particules polymères, un liant particulaire et une charge.

23. Trousse selon la revendication 11, dans laquelle la composition colorante se présente sous la forme d'une pâte ou d'une dispersion fluide, qui contient au moins un pigment coloré qui est lié aux particules polymères, un liant et une charge.

24. Procédé de fabrication de prothèses dentaires, **caractérisé en ce que** (i) on applique un pigment coloré, en surface, sur des particules polymères présentant une taille granulaire moyenne de 20 à 90 µm, (ii) on combine les particules polymères revêtues de pigment avec un liant et on forme une dispersion fluide ou pâteuse ou un comprimé, (iii) on détermine d'abord les quantités de compositions colorantes nécessaires à l'aide d'un nuancier et on mélange ensuite une ou plusieurs dispersions et/ou un ou plusieurs comprimés et/ou des parties de comprimés à un matériau de matrice polymérisable et à une charge, (iv) on forme le mélange pour obtenir une restauration dentaire ou une partie de celle-ci et (v) on durcit ensuite le tout.

25. Procédé selon la revendication 24, **caractérisé en ce que** l'on mélange d'abord, à l'étape (iii), le matériau de matrice polymérisable et la charge à une première composition colorante et **en ce que** l'on détermine ensuite la teinte de couleur du mélange en ajoutant une ou plusieurs autres compositions colorantes.

26. Utilisation d'une composition colorante selon l'une quelconque des revendications 1 à 23, pour réaliser la coloration individuelle de prothèses ou de parties de prothèses dentaires contenant une charge et présentant la couleur des gencives.
